(19) Europäisches Patentamt
European Patent Office
Office européen des brevets

(11) Numéro de publication: **0 251 874**
**A1**

(12) # DEMANDE DE BREVET EUROPEEN

(21) Numéro de dépôt: **87401410.3**

(22) Date de dépôt: **22.06.87**

(51) Int. Cl.$^4$: **C 12 N 15/00**
C 12 N 9/64

(30) Priorité: **23.06.86 FR 8609043**

(43) Date de publication de la demande:
**07.01.88 Bulletin 88/01**

(84) Etats contractants désignés:
**AT BE CH DE ES FR GB GR IT LI LU NL SE**

(71) Demandeur: **TRANSGENE S.A.**
**16, rue Henri Régnault**
**F-92400 Courbevoie (FR)**

(72) Inventeur: **De La Salle, Henri**
**10, rue du Général Leclerc**
**F-67400 Ostwald (FR)**

**Elkaim, René**
**9, place de Haguenau**
**F-67000 Strasbourg (FR)**

(74) Mandataire: **Warcoin, Jacques et al**
**Cabinet Régimbeau 26, avenue Kléber**
**F-75116 Paris (FR)**

(54) **Vecteurs d'integration dans les cellules eucaryotes assurant l'expression du facteur IX, lignées cellulaires obtenues et procédé pour leur préparation.**

(57) L'invention concerne un vecteur d'intégration dans les cellules eucaryotes, de séquences d'ADN assurant l'expression du facteur IX ou d'une protéine analogue dans lesdites cellules, caractérisé en ce qu'il comporte au moins :
- une séquence d'ADN codant pour le facteur IX ou la protéine analogue,
- les éléments assurant l'intégration sous forme du multicopie de ladite séquence,
- les éléments assurant, après intégration, l'expression, dans lesdites cellules, de la séquence d'ADN codant pour le facteur IX ou la protéine analogue.

**EP 0 251 874 A1**

**Description**

# VECTEURS D'INTEGRATION DANS LES CELLULES EUCARYOTES ASSURANT L'EXPRESSION DU FACTEUR IX, LIGNEES CELLULAIRES OBTENUES ET PROCEDE POUR LEUR PREPARATION

La présente invention concerne l'obtention de lignées cellulaires produisant constitutivement du facteur IX humain ou des molécules analogues.

La Demanderesse a décrit dans des brevets antérieurs, notamment le brevet WO 85 05376 et le brevet WO 8505125,des procédés permettant de transformer des cellules hôtes afin de les rendre capables de produire le facteur IX.

Le facteur IX, comme cela a déjà été mentionné dans ces brevets, est une protéine intervenant au cours du cycle de la coagulation du sang et qui est utilisée pour le traitement des hémophilies B. Pour l'instant, la seule source disponible de facteur IX est le plasma humain.

Les préparations de facteur IX obtenues par extraction à partir de plasma peuvent être pyrogènes et comportent également des risques de contamination par des agents pathogènes ou des virus, notamment le virus de l'hépatite ou les agents vecteurs du SIDA.

C'est pourquoi il est particulièrement intéressant de préparer le facteur IX avec une très haute pureté à partir de procédés qui ne mettent pas en jeu l'extraction à partir du plasma humain ou animal.

Les brevets mentionnés précédemment permettaient d'apporter une réponse partielle au problème évoqué précédemment, puisque ces brevets décrivent les procédés permettant de préparer le facteur IX à partir d'hôtes tels que les bactéries, les levures ou bien les cellules animales.

La présente invention a pour objet la mise au point de vecteurs d'un type différent de ceux qui dont déjà été décrits dans la mesure où il s'agit de vecteurs d'intégration permettant d'obtenir des lignées cellulaires stables produisant le facteur IX.

Plus particulièrement, il s'agit de vecteurs d'intégration dans les cellules eucaryotes. Ces vecteurs contiennent des séquences d'ADN assurant l'expression du facteur IX ou d'une protéine analogue dans lesdites cellules et caractérisés en ce qu'ils comportent au moins :

- une séquence d'ADN codant pour le facteur IX ou la protéine analogue,
- les éléments assurant l'intégration sous forme de multicopie de ladite séquence,
- les éléments assurant, après intégration, l'expression,dans lesdites cellules,de la séquence d'ADN codant pour le facteur IX ou la protéine analogue.

Par "vecteurs d'intégration", on entend désigner un ou plusieurs plasmides qui s'intègrent dans le génome des cellules eucaryotes en cause, de préférence ces cellules étant des cellules de mammifère.

De façon générale, on entendra désigner dans la présente description par "protéine analogue au facteur IX" une protéine ayant le même type d'activité biologique <u>in vivo</u> ou une activité apparentée à celle du facteur IX. Il peut s'agir, par exemple, d'une protéine ayant la même structure que le facteur IX avec quelques modifications au niveau de certains amino-acides, ou bien éventuellement il peut s'agir de fragments du facteur IX ayant l'activité essentielle de la molécule complète.

La séquence d'ADN codant pour le facteur IX a été décrite dans les brevets précédents et il convient de rappeler que la séquence d'ADN codant pour le facteur IX complète comporte, outre un peptide signal, une séquence de pré-protéine. Comme cela sera démontré dans les exemples, il est préféreable de prendre à titre de séquence d'ADN codant pour le facteur IX l'ensemble de la séquence, c'est-à-dire la séquence signal et la pré-protéine.En particulier, ces séquences de l'extrémité 5' comportent trois ATG et il ressort des exemples que la présence de ces trois ATG favorise l'expression du facteur IX, bien que leur présence simultanée ne soit pas indispensable.

Le procédé permettant la préparation d'un clone porteur du gène du facteur IX ne sera pas redécrit en détail, on pourra pour ce faire se reporter aux brevets mentionnés précédemment.

La séquence assurant l'intégration sous forme de multicopie de la séquence d'ADN codant pour le facteur IX peut être de nature diverse, mais il s'agira plus particulièrement d'une séquence d'ADN mitochondrial d'origine murine, en particulier de ce type de séquence portée par un plasmide dénommé pDelta.

Afin d'obtenir des cellules comportant un grand nombre de copies du gène du facteur IX, il est intéressant de prévoir, sur les vecteurs d'intégration, la présence d'un gène de résistance à un produit chimique, par exemple les gènes XGPRT ou DHFR qui confèrent, respectivement, la résistance à l'acide mycophénolique et au méthotréxate.

Ces différents gènes pourront comporter leurs propres éléments d'expression dans la cellule hôte, il s'agira, par exemple, de promoteurs d'origine virale tels que les promoteurs de SV40 ou d'adénovirus, par exemple le promoteur majeur tardif de l'adénovirus 2 ou le promoteur précoce de SV40. Toutefois, dans certains types d'intégration, ces gènes pourront se trouver directement sous la dépendance de promoteurs chromosomiques, ce qui pourra écarter l'utilisation de promoteurs dans le vecteur plasmidique.

Ces gènes, notamment le gène du facteur IX, seront complétés de préférence à leur extrémité 3' par un intron et un site de polyadénylation, il pourra s'agir, par exemple, de l'intron 1 de la beta-globine de lapin et du signal de polyadénylation de l'antigène T du virus SV40.

Dans la mesure où les fragments d'ADN qui doivent être introduits dans le vecteur sont d'assez grande dimension, il peut, dans certains cas, être nécessaire de dédoubler le vecteur d'intégration ; dans ce cas, le vecteur d'intégration n'est pas contitué par un plasmide mais par deux plasmides qui comportent des

éléments d'intégration et dont l'un porte au moins le gène de résistance à un produit chimique et dont l'autre comporte le gène codant pour le facteur IX ou une protéine analogue.

Dans un autre mode de réalisation des vecteurs d'intégration selon la présente invention, le vecteur est conçu pour conduire à un mARN polycystronique, vecteur comportant, dans ce cas, un séquence d'ADN codant pour le facteur IX ou la protéine analogue suivie d'un gène de résistance à un produit chimique. Dans ce cas, comme cela a déjà été démontré à plusieurs reprises, les ribosomes peuvent ré-initier la traduction en aval d'un codon stop, ce qui permet de construire des plasmides contenant des blocs de sélection polycystroniques avec un promoteur, un gène du facteur IX, un gène de sélection, un intron et un site de polyadénylation.

La présente invention concerne également les procédés de préparation de cellules eucaryotes afin d'obtenir des lignées cellulaires produisant le facteur IX ou une protéine analogue, caractérisés en ce que l'on transfecte ces cellules par un vecteur d'intégration selon l'invention, ce vecteur d'intégration pouvant être constitué par un plasmide unique ou bien par un couple de plasmides comme cela a été décrit précédemment.

Parmi les méthodes de transfection utilisées, il faut citer tout particulièrement la technique de co-précipitation au phosphate de calcium, qui sera mise en oeuvre dans les exemples ci-après.

Enfin, l'invention concerne les lignées cellulaires obtenues par la mise en oeuvre du procédé selon la présente invention et tout particulièrement les lignées CHO qui produisent le facteur IX,ainsi qu'un procédé de préparation du facteur IX dans lequel on cultive ces cellules sur un milieu de culture approprié et on récupère le facteur IX à partir de la culture.

Enfin, la présente invention concerne le facteur IX obtenu par la mise en oeuvre du procédé décrit précédemment.

Les exemples ci-après sont destinés à mettre en évidence d'autres caractéristiques et avantages de la présente invention.

Les exemples seront décrits en se référant aux figures sur lesquelles:

. la figure 1 représente la construction de pTG395,
. la figure 2 représente la construction de pTG385,
. la figure 3 représente la construction de pTGDH386,
. la figure 4 représente la construction de pTG384,
. la figure 5 représente la construction de pTG381.

Sauf indication contraire, les différents moyens utilisés dans les exemples, enzymes par exemple, sont utilisés dans les conditions préconisées par le fournisseur.

Vecteurs

Les vecteurs dérivent du plasmide pDelta (G. Lutfalla et coll. Somatic Cell and Mol. Genet. 11, 223-238, 1983). Ce plasmide contient une séquence de l'ADN mitochondrial murin permettant au plasmide de s'intégrer dans le génome des cellules de mammifères en plusieurs centaines de copies répétées. Ce plasmide possède par ailleurs le gène bactérien de la xanthine-guanine phosphoribosyl-transféranse (XGPRT) sous le contrôle du promoteur précoce de SV40 ; il peut être utilisé comme marqueur de sélection pour la résistance à l'acide mycophénolique.

EXEMPLE 1

SUBSTITUTION DU GENE XGPRT A UN BLOC D'EXPRESSION DU FACTEUR IX

Le fragment EcoRI-BamHI de pSVBIA34 (R. Hen et coll. PNAS 79, 7132-7136) contenant la séquence activatrice de SV40 et une partie du promoteur majeur tardif de l'adénovirus 2 est cloné dans le vecteur m13TG127 entre les sites EcoRI et BamHI donnant le clone mTG394. L'ADN de mTG394 est digéré par EcoRI et BglII puis cloné dans pTG157, coupé par EcoRI et BglII substituant ainsi le promoteur TK par le promoteur hybride (pTG395).

pTG395 est ensuite digéré par EcoRI et SalI et le bloc d'expression (promoteur, intron, site de polyadénylation) est cloné dans pDelta digéré par EcoRI et SalI, éliminant ainsi le gène de sélection XGPRT (pTG387). Le cADN du facteur IX provenant de la digestion partielle par XhoII de mTG312, contenant la séquence codante entière du cADN du facteur IX et une partie de la séquence non codante 3′ du cADN jusqu'au deuxième site XhoII du cADN, est cloné dans le site BamHI de pTG387 (pTG386). Le plasmide pTG385 est obtenu par insertion dans le site BamHI de pTG387 du fragment BamHI de mTG315.

Deux différences existent entre pTG386 et pTG385. La partie 3′ non codante est plus longue chez pTG385 que chez pTG386, mais surtout les extrémités 5′ sont différentes :
ainsi la séquence de l'extrémité 5′ de pTG385 est :
GAT C ATG CAG CGC GTG AAC ATG ATC ATG ... et celle de pTG386 est : GATC C GTC CTG GAA C ATG ATC ATG. Ces séquences peuvent être comparées à celle du cADN du facteur IX : ATG CAG CGC GTG AAC ATG ATC ATG ...

Une représentation schématique de ces constructions est donnée à la figure 1.

Ainsi pTG385 code pour le cADN entier alors que pTG386 permet l'initiation de la traduction seulement à partir du deuxième codon ATG. Ces deux constructions peuvent donc montrer lequel des codons iniateurs de la traduction doit être utilisé pour obtenir une production optimale du facteur IX.

Ces plasmides ont ensuite été transférés dans les cellules CHO. A cette fin, 10 μg de pTG385 (ou pTG386)

ont été mélangés avec 0,1 µg de plasmide pdelta puis ont été coprécipités en utilisant la technique classique du coprécipité de calcium. Dans ces expériences, le précipité est réalisé en l'absence d'ADN entraîneur ; 48 heures après transfert dans les cellules, celles-ci sont trypsinées et réparties dans de nouvelles boîtes afin de les diluer au 1/5ème. Le milieu de sélection est utilisé à partir de ce moment :
milieu $alpha_{2000}$ supplémenté en hypoxanthine (15 mg/l), thymidine (10 mg/l), xanthine (250 mg/l), aminoptérine (20 mg/l), acide mycophénolique (25 mg/l) et sérum de veau foetal dialysé 10 %.

La production de facteur IX (en ng) pour $10^6$ cellules et en 24 heures a ensuite été déterminée pour chacun des clones isolés.

Les résultats sont consignés dans le tableau suivant :

| Quantité de facteur IX produit (ng/$10^6$ cellules/24 h) | | 0 | 0-10 | 10-20 | 20-30 | 30-40 | 40-50 |
|---|---|---|---|---|---|---|---|
| Nombre de clones produisant du facteur IX | pTG386 | 4 | 0 | 3 | 1 | 0 | 0 |
| | pTG385 | 5 | 0 | 2 | 0 | 0 | 1 |

| Quantité de facteur IX produit (ng/$10^6$ cellules/24 h) | | 50-60 | 60-70 | 70-80 | 80-90 | 90-100 | 100-110 |
|---|---|---|---|---|---|---|---|
| Nombre de clones produisant du facteur IX | pTG386 | 0 | 0 | 0 | 0 | 0 | 0 |
| | pTG385 | 0 | 1 | 0 | 0 | 0 | 2 |

Deux conclusions peuvent être tirées :

1) Les vecteurs dérivés de pDelta permettent d'obtenir d'emblée des niveaux d'expression de 100 ng/$10^6$ cellules/24 heures.

2. Il est préférable d'utiliser la séquence entière du cADN du facteur IX plutôt que de la tronquer du premier ATG initiateur.

EXEMPLE 2

Il a été démontré à plusieurs reprises que les ribosomes peuvent réinitier la traduction en aval de codon stop. Cette propriété peut être utilisée pour construire des plasmides contenant un bloc de sélection polycystronique : promoteur, gène du facteur IX, gène de sélection, intron, site de polyadénylation. Deux gènes de sélection ont été employés : le gène de souris codant pour la dihydrofolate réductase (DHFR) et le gène bactérien de la XGPRT.

a) PLASMIDE CODANT POUR UN MESSAGER POLYCYSTRONIQUE CONSTITUE DES SEQUENCES CODANTES DU FACTEUR IX ET DE LA DHFR

Un fragment HindIII-BglII contenant le cADN de la DHFR de souris a été isolé de pMTVdhfr (Lee et coll., Nature 294, 228-232, 1981) puis cloné entre les sites correspondants de pTG386. Le recombinant obtenu est dénoté pTGDH386 et contient une unité transcriptionnelle constituée par la séquence activatrice du virus SV40 couplée au promoteur majeur tardif de l'adénovirus 2, le cADN du facteur IX, le cADN de la DHFR de souris, un intron du gène de la beta-globine de lapin et le signal de polyadénylation de l'antigène T du virus SV40. On a transféré le recombinant pTGDH386 dans des cellules CHO ($DHFR^-$) par la technique de coprécipitation de l'ADN au phosphate de calcium. On a utilisé 5 µg de pTGDH386 par boîte de Petri de diamètre 10 cm, contenant $5.10^5$ cellules. Après 24 heures de mise en contact du précipité d'ADN avec les cellules, le milieu est aspiré, les cellules lavées par du PBS et le milieu sélectif $alpha_{2000}$ supplémenté en sérum foetal dialysé à 10 % est ajouté aux cellules. Après un mois de sélection, on reprend 25 clones et la quantité de facteur IX produite est déterminée à l'aide du test ELISA.

On a condensé les résultats dans le tableau suivant où la quantité de facteur IX est exprimé en ng sécrété dans le milieu par 24 heures et pour $10^6$ cellules.

| Quantité de facteur IX sécrétée | 0 | 0-20 | 20-40 | 40-60 | 60-80 | 80-100 |
|---|---|---|---|---|---|---|
| Nombre de clones produisant le facteur IX | 6 | 11 | 5 | 2 | 1 | 1 |

La quantité de facteur IX produite est plus faible que celle attendue au regard des résultats obtenus avec les recombinants pTG381 et pTG382. Ce point peut s'expliquer par une plus forte pression de sélection de la XGPRT nécessitant alors un nombre plus important de copies intégrées du plasmide afin d'acquérir la résistance à l'acide mycophénolique. Dans le cas de la DHFR (pTGDH386) un nombre plus restreint de copies intégrées au génome pourraît être suffisant pour conférer aux clones la capacité de croître sur milieu sélectif. Une seconde explication peut résider dans la perte de séquences contenant le premier ATG supposé initiateur de la traduction du cADN du facteur IX (voir pTG385 et pTG386). La perte de ces séquences pourraît considérablement affecter le niveau général de traduction du messager polycistronique issu de pTGDH386. En effet, au vu des résultats mentionnés précédemment avec pTG385 et pTG386, une différence entre ces deux recombinants est observée dans le niveau général d'expression du facteur IX. L'obtention de clones CHO capables de croître en milieu sélectif et contenant un messager polycistronique constitué du cADN complet du facteur IX suivi du cADN de la DHFR, pourraît apporter un élément de réponse sur le rôle des séquences initiatrices situées en 5′ du cADN du facteur IX.

b) PLASMIDES POLYCISTRONIQUES FACTEUR IX - XGPRT

Le promoteur du gène XGPRT de pDelta a d'abord été substitué par celui de l'adénovirus 2 déjà décrit. A cette fin, pDelta a été digéré par les enzymes HindIII et NruI et ligué avec le fragment PvuII-HindIII de pTG395 contenant le bloc promoteur (pTG384). Ensuite, pTG384 a été digéré partiellement par BamHI et le fragment linéarisé de grande taille a été purifié sur gel d'agarose puis ligué avec le fragment BamHI de mTG315 (donnant le plasmide pTG382).

Une autre construction a été réalisée avec un cADN possédant une séquence 5′ non traduite différente. Pour réaliser cette construction le fragment FnuDII-PstI du cADN du facteur IX contenu dans pTG397 a été isolé puis cloné dans le vecteur m13TG120 entre les sites PstI et SalI, en utilisant l'adaptateur SalI FnuDII :

T C GAC CATG CAG CG G GTAC GTC GC

restaurant ainsi la séquence codante entière du cADN du facteur IX (mTG390). Le cADN du facteur IX a été ensuite isolé de mTG390 sous forme de fragment BamHI/XhoII partiel de 1800 bp environ puis cloné dans le site BamHI de pTG186 (pTG391). Le cADN du facteur IX a ensuite été extrait de pTG391 sous forme de fragment BamHI puis a été cloné dans pTG386 coupé partiellement par BamHI. Un clone susceptible de coder pour un ARN polycistronique facteur IX-XGPRT a pu ainsi être isolé (pTG381). La construction pTG381 a été réalisée pour rendre plus conforme l'extrémité 5′ non codante aux règles émises par M. Kozak (M. Kozak, Nucl. Acids Res. 12, 857-872, 1984).

Les deux plasmides pTG381 et pTG382 ont ensuite été transférés dans les cellules CHO par la technique classique du coprécipité de phosphate de calcium à raison de 5 µg ou de 10 µg de plasmide par boîte de Petri de 10 cm de diamètre conenant un tapis de cellules CHO 50 % confluentes.

Les clones ont ensuite été sélectionnés en utilisant la technique décrite dans l'exemple 1.

Environ 36 clones ont été sélectionnés et analysés (23 séries pTG831, 13 séries pTG382). Environ 25 % d'entre eux produisent plus de 1 µg pour $10^6$ cellules en 24 heures. Le facteur IX dans les surnageants de deux clones, 571 (CHO transfecté avec pTG381) et 742 (CHO transfecté avec pTG382) a été titré par test ELISA et test d'activité. Pour cette titration des tapis de cellules 75 % confluentes ont été placés dans le milieu $alpha_{2000}$ supplémenté en sérum foetal inactivé 30 minutes à 55° C et adsorbés sur hydroxyde d'alumine. Après 15 heures le milieu a été récolté et titré et les cellules furent trypsinées et comptées.

Les résultats sont les suivants :

571 produit 3,84 µg de facteur IX pour $10^6$ cellules en 24 heures, actif à 14 %,
741 produit 1,4 µg de facteur IX pour $10^6$ cellules en 24 heures, actif à 13 %.

En conclusion, ces vecteurs permettent d'obtenir des clones possédant un niveau d'expression élevé. Les cellules CHO sont capables d'effectuer au moins en partie des modifications post-traductionnelles essentielles pour l'activité du facteur IX : 13 % du facteur IX produit est actif, ou l'activité du facteur IX est

équivalente à 13 % de celle du facteur IX humain. Les résultats de transfection avec pTG381 et pTG382 montrent qu'il peut être préférable d'améliorer la région 5' non codante au voisinage du premier ATG de façon à améliorer l'initiation de la traduction du mARN codant pour le facteur IX.

Les souches suivantes ont été déposées le 6 juin 1986 à la Collection Nationale de Cultures de Microorganismes de l'Institut pasteur, 28 rue du Docteur-Roux, 75724 Paris Cédex 15 :

. E. coli BJ5183/pTG385, sous le nº I-563

. E. coli BJ5183/pTG381, sous le nº I-564.

**Revendications**

1) Vecteur d'intégration, dans les cellules eucaryotes, de séquences d'ADN assurant l'expression du facteur IX ou d'une protéine analogue dans lesdites cellules, caractérisé en ce qu'il comporte au moins :
- une séquence d'ADN codant pour le facteur IX ou la protéine analogue,
- les éléments assurant l'intégration sous forme de multicopie de ladite séquence,
- les éléments assurant, après intégration, l'expression, dans lesdites cellules, de la séquence d'ADN codant pour le facteur IX ou la protéine analogue.

2) Vecteur selon la revendication 1, caractérisé en ce qu'il comporte au moins :
- une séquence d'ADN codant pour le facteur IX ou la protéine analogue,
- une séquence assurant l'intégration sous forme multicopie de ladite séquence d'ADN,
- les éléments d'expression de ladite séquence d'ADN dans lesdites cellules.

3) Vecteur selon la revendication 1, caractérisé en ce que la séquence d'ADN codant pour le facteur IX comportant la séquence signal est la séquence de pré-protéine complète.

4) Vecteur selon l'une des revendications 2 et 3, caractérisé en ce que la séquence assurant l'intégration sous forme multicopie de ladite séquence d'ADN est une séquence d'ADN mitochondrial murin.

5) Vecteur selon la revendication 4, caractérisé en ce que la séquence d'ADN mitochondrial murin est celle de pDelta.

6) Vecteur selon l'une des revendications 1 à 5, caractérisé en ce qu'il comporte un gène de résistance à un produit chimique ainsi que les éléments assurant son expression dans les cellules transformées.

7) Vecteur selon l'une des revendications 1 à 6, caractérisé en ce qu'il code pour un ARN polycistronique comportant la séquence d'ADN codant pour le facteur IX ou la protéine analogue suivie d'un gène de résistance à un produit chimique.

8) Vecteur selon l'une des revendications 6 et 7, caractérisé en ce que le gène de résistance est choisi parmi le gène codant pour la XGPRT et la DHFR.

9) Vecteur selon l'une des revendications 1 à 8, caractérisé en ce que les éléments assurant l'expression des séquences d'ADN ou des gènes sont choisis parmi les promoteurs de SV40 et de l'adénovirus 2.

10) Vecteur selon l'une des revendications 1 à 9, caractérisé en ce que le gène du facteur IX est suvi d'un intron et d'un site de polyadénylation.

11) Vecteur selon l'une des revendications 1 à 10, caractérisé en ce qu'il est constitué d'un seul plasmide portant l'ensemble des séquences d'ADN.

12) Vecteur selon l'une des revendications 1 à 10, caractérisé en ce qu'il est constitué d'un couple de plasmides comportant des éléments assurant l'intégration et dont l'un contient au moins la séquence d'ADN codant pour le facteur IX ou la protéine analogue et l'autre contient au moins un gène de sélection.

13) Procédé de préparation de cellules eucaryotes afin d'obtenir des lignées cellulaires produisant le facteur IX ou une protéine analogue, caractérisé en ce qu'on effectue la transfection de ces cellules par un vecteur selon l'une des revendications 1 à 12 et que l'on sélectionne les cellules exprimant le facteur IX ou la protéine analogue.

14) Procédé selon la revendication 13, caractérisé en ce que la transfection est effectuée par la technique de coprécipitation au phosphate de calcium.

15) Lignée cellulaire obtenue par la mise en oeuvre du procédé selon l'une des revendications 14 et 14.

16) Lignée cellulaire selon la revendication 15, caractérisée en ce qu'il s'agit d'une lignée CHO.

17) Procédé de préparation de facteur IX ou d'une protéine analogue, caractérisé en ce qu'on cultive une lignée cellulaire selon l'une des revendications 15 et 16 sur un milieu de culture approprié et en ce qu'on récupère le facteur IX à partir de la culture.

18) Facteur IX obtenu par la mise en oeuvre du procédé selon la revendication 17.

0251874

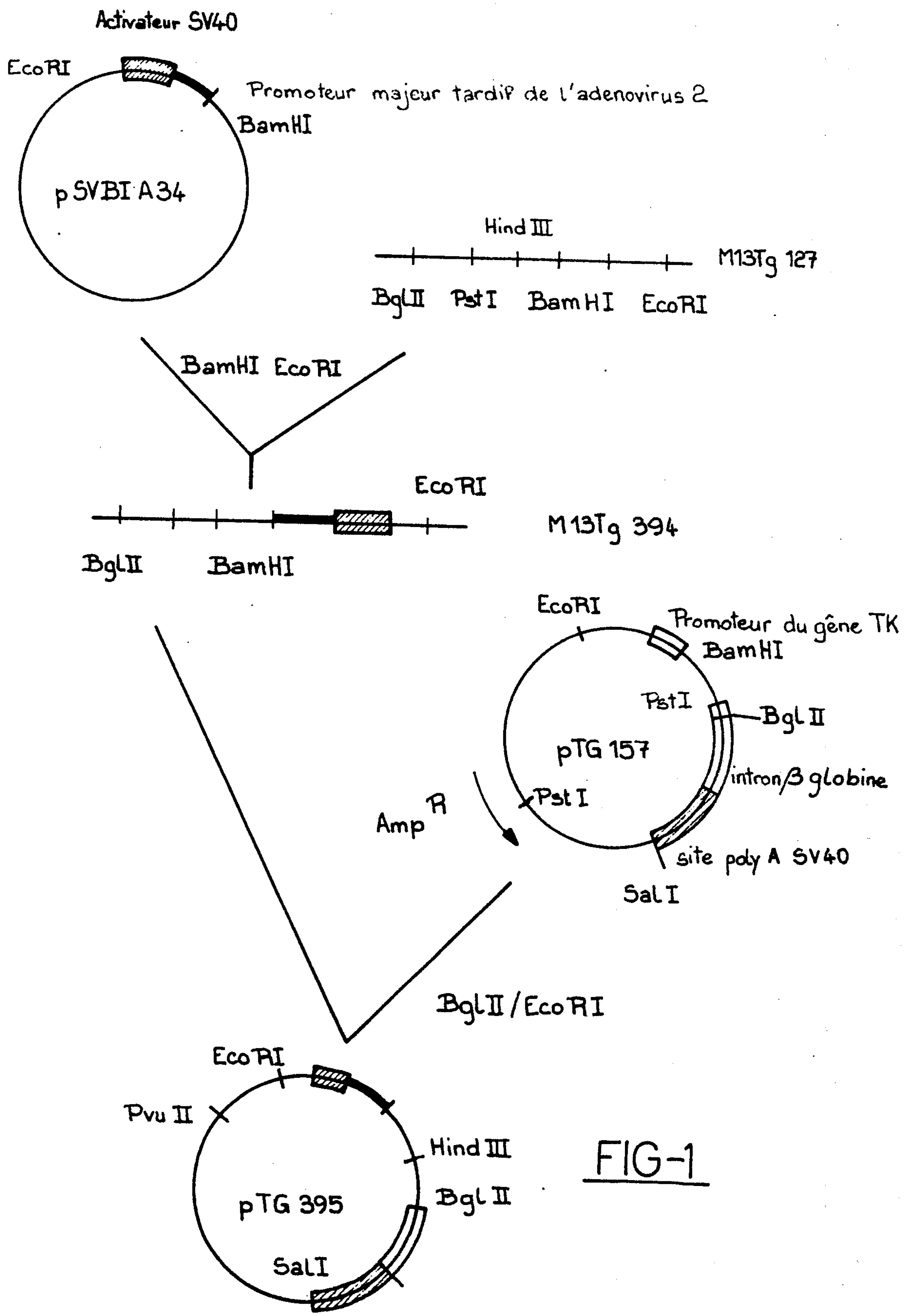


FIG-1

0251874

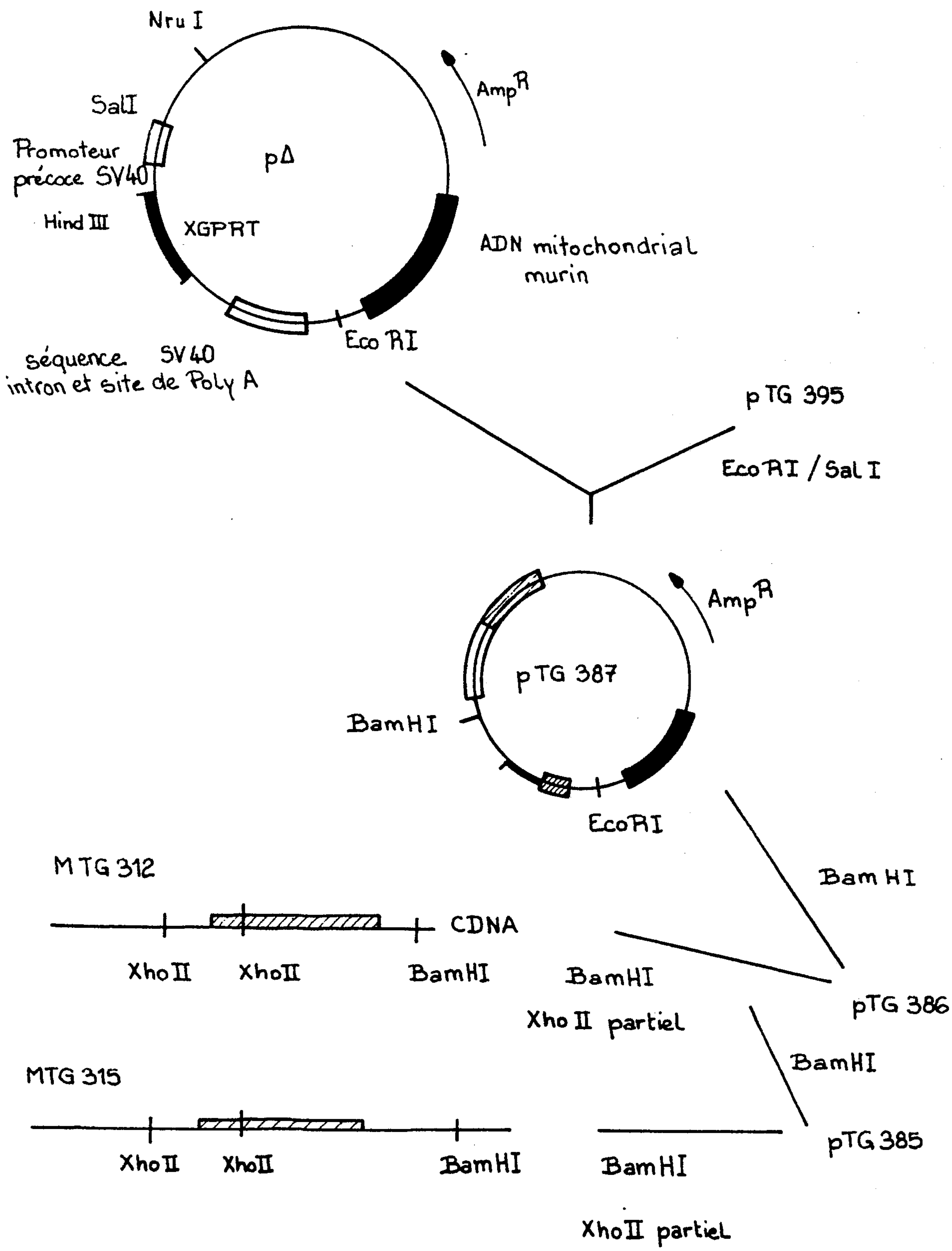


FIG-2

0251874

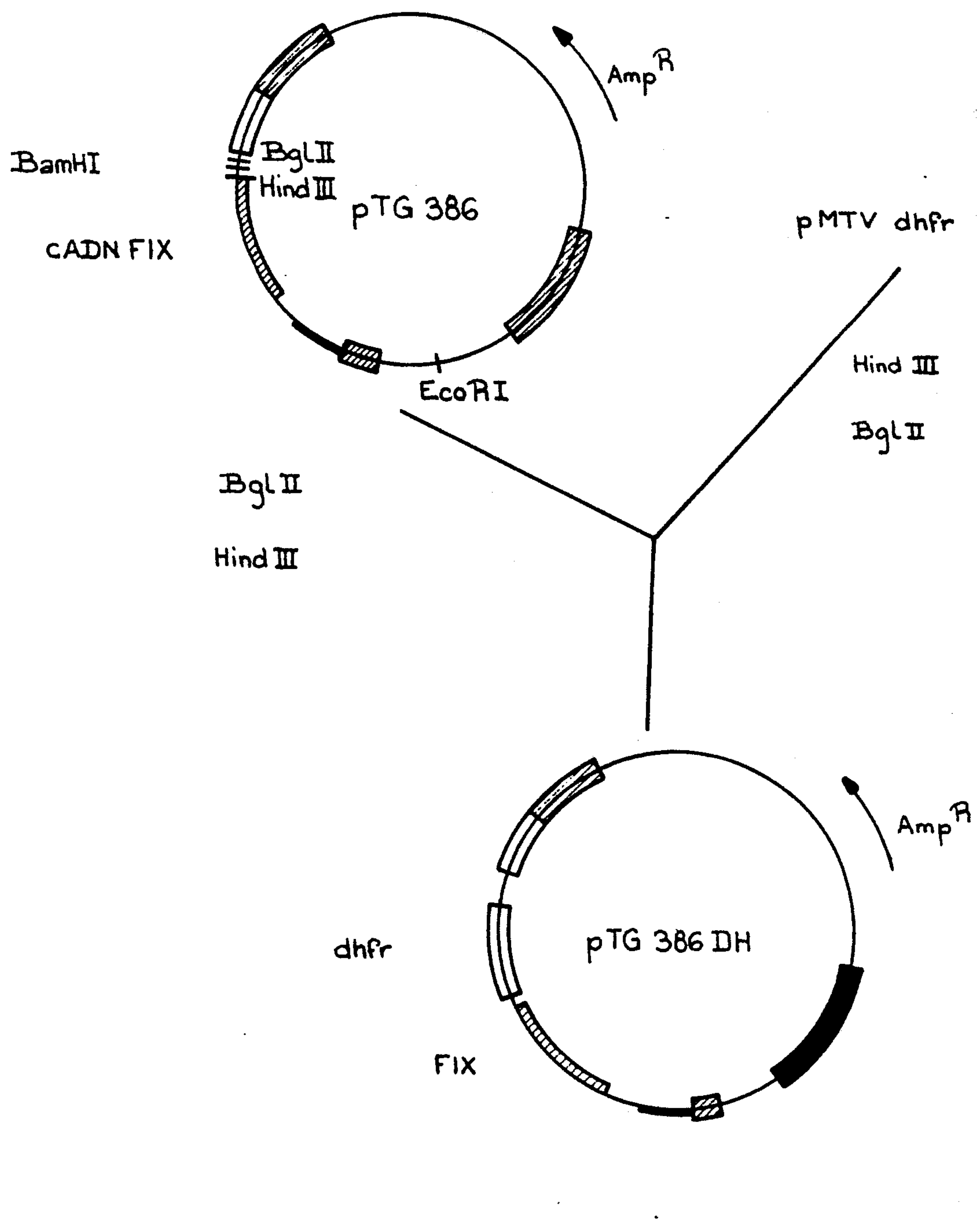


FIG-3

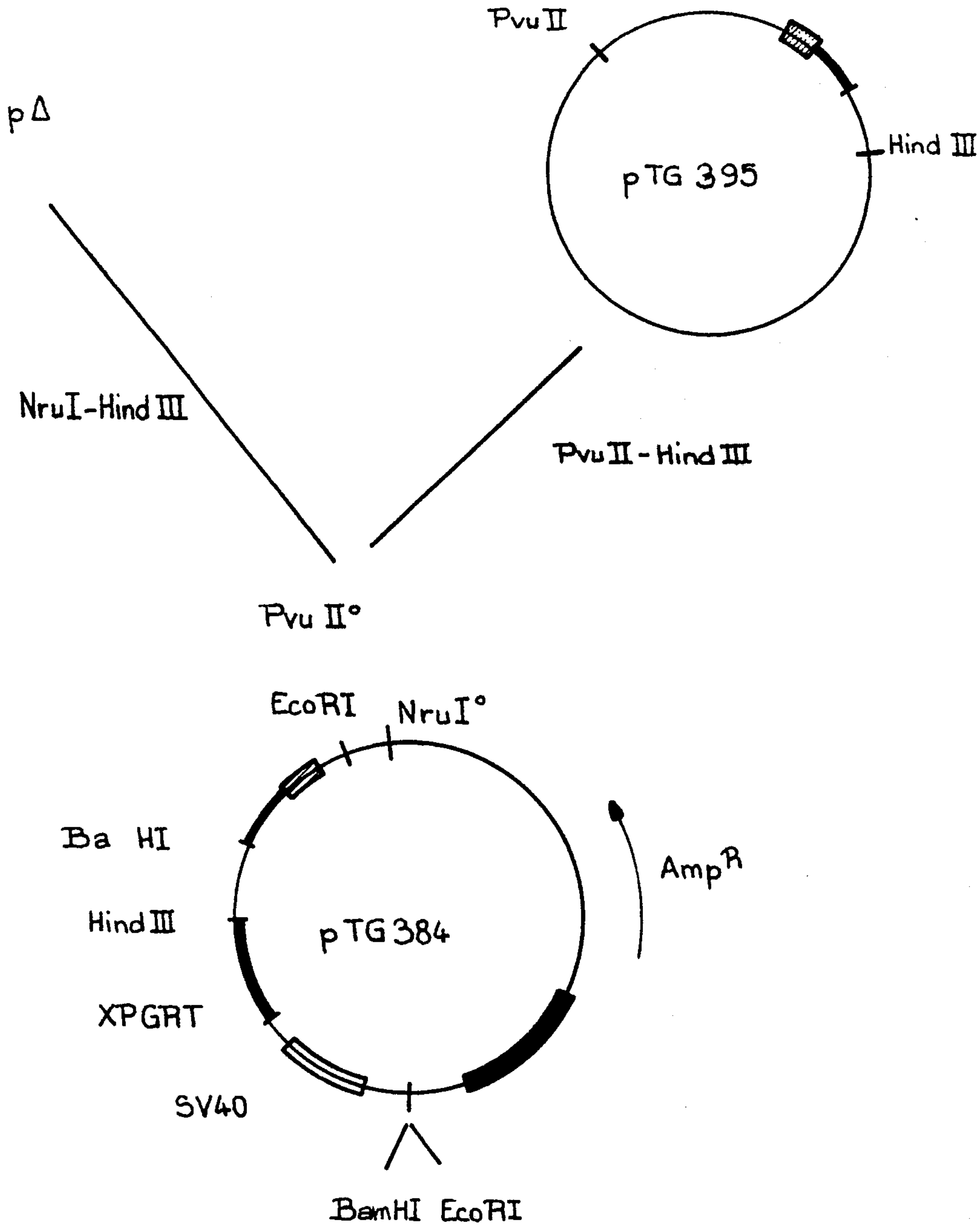
pΔ
PvuII
pTG 395
Hind III
NruI-Hind III
PvuII-Hind III
PvuII°
EcoRI
NruI°
Ba HI
Hind III
pTG 384
AmpR
XPGRT
SV40
BamHI EcoRI

FIG-4

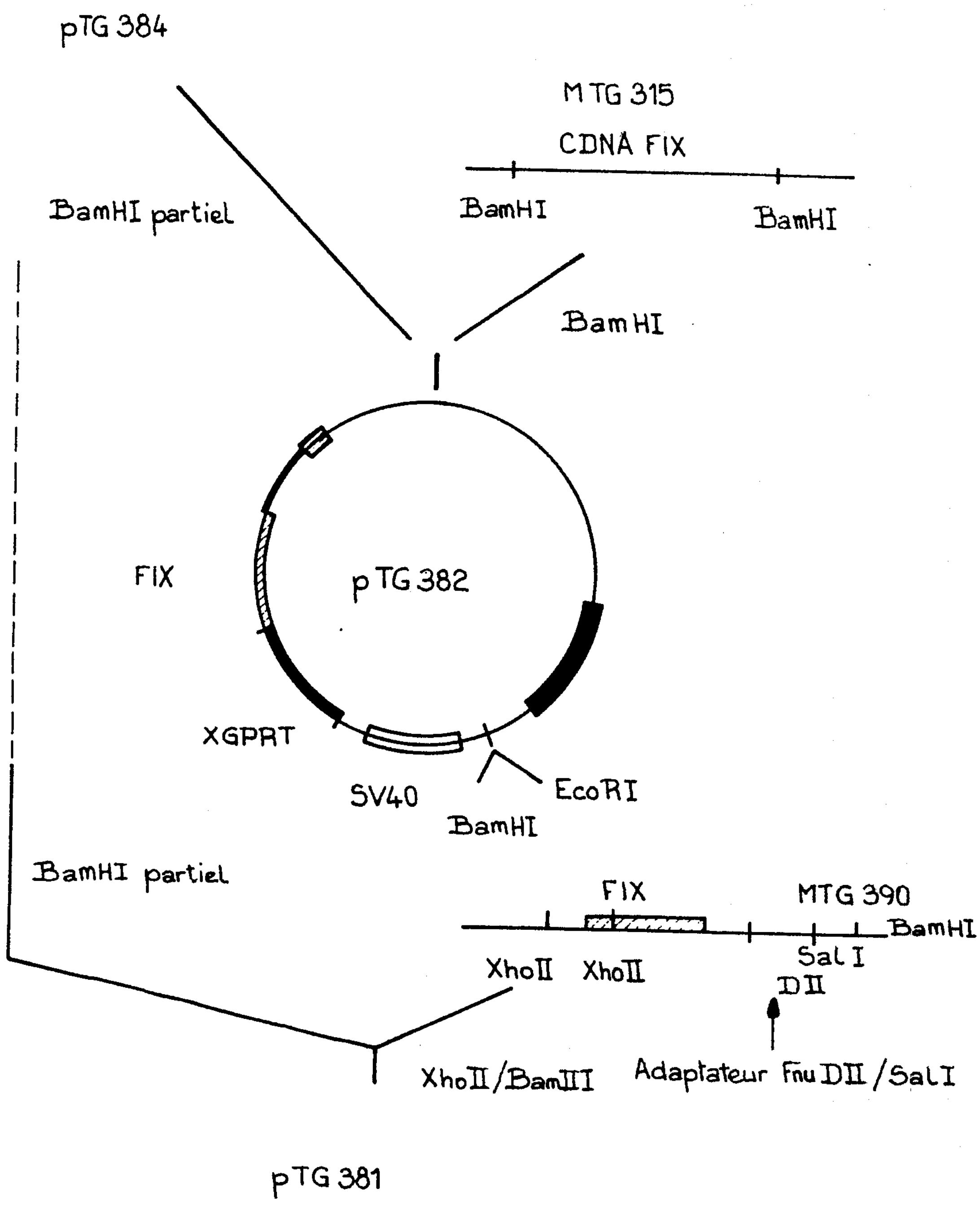
pTG 384
MTG 315
CDNA FIX
BamHI partiel
BamHI
BamHI
BamHI
FIX
pTG 382
XGPRT
SV40
EcoRI
BamHI
BamHI partiel
FIX
MTG 390
BamHI
XhoII
XhoII
Sal I
DII
XhoII/BamHI
Adaptateur FnuDII/SalI
pTG 381

FIG-5

Office européen des brevets

# RAPPORT DE RECHERCHE EUROPEENNE

Numéro de la demande

EP 87 40 1410

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | BLOOD, JOURNAL HEMATOLOGY, novembre 1985, supplément, page 343a, résumé no. 1256; C. SHOEMAKER et al.: "Expression, purifcation and characterization of biologically active human factor IX synthesized by recombinant mammalian host cells" * Résumé * | 1,5,8, 13-18 | C 12 N 15/00<br>C 12 N 9/64 |
| Y | NATURE, vol. 315, no. 6021, 20 juin 1985, pages 683-685, Londres, GB; D.S. ANSON et al.: "Expression of active human clotting factor IX from recombinant DNA clones in mammalian cells" * En entier * | 1,2 | |
| | | | **DOMAINES TECHNIQUES RECHERCHES (Int. Cl.4)** |
| A | Idem | 6,13-15,17, 18 | C 12 N |
| Y | NATURE, vol. 316, no. 6025, 18 juillet 1985, pages 268-270, Londres, GB; H. DE LA SALLE et al.: "Active gamma-carboxylated human factor IX expressed using recombinant DNA techniaues * En entier * | 1,2 | |

-/-

Le présent rapport de recherche a été établi pour toutes les revendications

| Lieu de la recherche | Date d'achèvement de la recherche | Examinateur |
|---|---|---|
| LA HAYE | 30-07-1987 | YEATS S.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82

Office européen des brevets

# RAPPORT DE RECHERCHE EUROPEENNE

Numéro de la demande

EP 87 40 1410



## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | Idem<br>--- | 13 | |
| Y | NATURE, vol. 316, no. 6025, 18 juillet 1985, pages 271-273, Londres, GB; S. BUSBY et al.: "Expression of active human factor IX in transfected cells"<br>* En entier * | 1,2 | |
| A | Idem<br>--- | 6,9,13 | |
| Y | EP-A-0 107 278 (NATIONAL RESEARCH DEVELOPMENT CORP.)<br>* Page 6, ligne 19 - page 7, ligne 23; revendications *<br>--- | 1,2 | |
| A | PROCEEDINGS OF THE NATIONAL ACADEMY OF SCIENCES OF THE USA, vol. 79, no. 21, novembre 1982, pages 6461-6464, Washington, US; K. KURACHI et al.: "Isolation and characterization of a cDNA coding for human factor IX"<br>* Résumé *<br>--- | 1 | DOMAINES TECHNIQUES RECHERCHES (Int. Cl.4) |
| P,X | WO-A-8 606 408 (GENETICS INSTITUTE, INC.)<br>* Page 3, lignes 5-21; exemples 7,8; revendications *<br>--- -/- | 1,2,6, 8,9,13 -18 | |

Le présent rapport de recherche a été établi pour toutes les revendications

| Lieu de la recherche | Date d'achèvement de la recherche | Examinateur |
|---|---|---|
| LA HAYE | 30-07-1987 | YEATS S.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82

Office européen des brevets

**RAPPORT DE RECHERCHE EUROPEENNE**

Numéro de la demande

EP 87 40 1410



## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| P,X | THE JOURNAL OF BIOLOGICAL CHEMISTRY, vol. 261, no. 21, 25 juillet 1986, pages 9622-9628, American Scoety of Biological Chemists, Inc., US; R.J. KAUFMAN et al.: "Expression, purification, and characterization of recombinant gamma-carboxylated factor IX synthesized in chinese hamster ovary cells" * Résumé; pages 9624, colonne 1, lignes 5-27; table 1 * --- | 1,2,6,8,9,13-18 | |
| P,X | EP-A-0 195 592 (NATIONAL RESEARCH DEVELOPMENT CORP.) * Page 5, lignes 11-22; page 7, lignes 13-35; page 24, lignes 15-32; revendications * --- | 1,2,13,17,18 | |
| P,X | EP-A-0 200 421 (ZYMOGENETICS, INC.) * Page 5, lignes 21-40; exemple 7; table 4 * --- -/- | 1,2,6,7,9,13-15,17,18 | DOMAINES TECHNIQUES RECHERCHES (Int. Cl.4) |

Le présent rapport de recherche a été établi pour toutes les revendications

| Lieu de la recherche | Date d'achèvement de la recherche | Examinateur |
|---|---|---|
| LA HAYE | 30-07-1987 | YEATS S.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

Office européen des brevets

# RAPPORT DE RECHERCHE EUROPEENNE

Numéro de la demande

EP 87 40 1410



| DOCUMENTS CONSIDERES COMME PERTINENTS | | | |
|---|---|---|---|
| Catégorie | Citation du document avec indication, en cas de besoin, des parties pertinentes | Revendication concernée | CLASSEMENT DE LA DEMANDE (Int. Cl.4) |
| P,X | NUCLEIC ACIDS RESEARCH, vol. 15, no. 3, février 1987, pages 871-884, IRL Press Ltd, Oxford, GB; K.H. CHOO et al.: "Expression of active human blood clotting factor IX in transgenic mice: use of a cDNA with complet mRNA sequence" * Résumé; page 875, lignes 12-36; page 881, lignes 17-32 * <br> ----- | 1,2,6-8,13-15,17,18 | |
| | | | DOMAINES TECHNIQUES RECHERCHES (Int. Cl.4) |

Le présent rapport de recherche a été établi pour toutes les revendications

| Lieu de la recherche | Date d'achèvement de la recherche | Examinateur |
|---|---|---|
| LA HAYE | 30-07-1987 | YEATS S.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03.82